(19) Europäisches Patentamt
European Patent Office
Office européen des brevets

(11) Publication number: **0 115 660 A1**

(12) # EUROPEAN PATENT APPLICATION

(21) Application number: 83300413.8

(22) Date of filing: 26.01.83

(51) Int. Cl.[3]: C 07 C 118/06

(43) Date of publication of application: 15.08.84 Bulletin 84/33

(84) Designated Contracting States: CH DE FR GB IT LI NL

(71) Applicant: INDIAN EXPLOSIVES LIMITED, ICI House 34 Chowringhee Road, Calcutta-700 071 West Bengal (IN)
Applicant: CHEMICALS AND FIBRES OF INDIA LIMITED, Crescent House 19 Walchand Hirachand Marg, Bombay-400 038 Maharashtra (IN)

(72) Inventor: Bhaduri, Sumit Alchemie Research Centre, Private Limited Cafi Site P.O. Box 155, Belapur Road Thane-400601 Maharashtra (IN)
Inventor: Sharma, K. R. Alchemie Research Centre, Private Limited Cafi Site P.O. Box 155, Belapur Road Thane-400601 Maharashtra (IN)
Inventor: Gopalkrishnan, K. Alchemie Research Centre, Private Limited Cafi Site P.O. Box 155, Belapur Road Thane-400601 Maharashtra (IN)

(74) Representative: Collier, Jeremy Austin Grey et al, J.A.Kemp & Co. 14, South Square Gray's Inn, London WC1R 5EU (GB)

(54) **Process for the preparation of isocyanates.**

(57) Isocyanates, or derivatives thereof, are produced from nitrobenzene or 2,4-dinitrotoluene by reaction with carbon monoxide in the presence of, as catalyst, an iron, ruthenium, rhodium, or platinum carbonyl.

EP 0 115 660 A1

DESCRIPTION

"PROCESS FOR THE PREPARATION OF ISOCYANATES"

The present invention relates to the preparation of isocyanates or derivatives of isocyanates and more particularly to the preparation of isocyanates or derivatives thereof by direct carbonylation of organic nitro compounds.

According to the present invention, a process for the preparation of isocyanates or derivatives thereof comprises reacting nitrobenzene or 2,4-dinitrotoluene with carbon monoxide, or a mixture of carbon monoxide and inert gas, in the presence of, as catalyst, a polynuclear metal carbonyl or derivative thereof of the formula $M_x(CO)_yL_z$, $^{n-}$, wherein

| | | | | | |
|---|---|---|---|---|---|
| M = Fe | x = 3 | y = 12 | z = 0 | n = 0 | |
| Fe | x = 3 | y = 11 | z = 1 | L = H | n = 1 |
| Ru | x = 3 | y = 12 | z = 0 | n = 0 | |
| Ru | x = 3 | y = 11 | z = 1 | L = H | n = 1 |
| Ru | x = 4 | y = 12 | z = 4 | L = H | n = 0 |
| Rh | x = 6 | y = 16 | z = 0 | n = 0 | |
| Pt | x = 15 | y = 30 | z = 0 | n = 2 | |

The reaction may be represented as follows:

$$RNO_2 + 3CO \longrightarrow RNCO + 2CO_2$$

where R is phenyl or the residue of 2,4-dinitrotoluene.

This reductive carbonylation reaction has two distinct advantages over the usual two step isocyanate synthesis based on the use of phosgene. First, it avoids the use of highly toxic phosgene and the formation of corrosive hydrochloric acid. Second, the hydrogenation step to convert the nitro compound to the corresponding amine derivative of the conventional

process can be avoided, and the isocyanate is obtained in a single step.

Although in the prior art there are examples of transition metal complex catalysed direct carbonylation of nitro compounds, the present process, which employs a novel class of metal complexes as catalysts, operates at a much reduced pressure with comparable yields and selectivity.

The process involves reaction of the organic nitro compound with carbon monoxide, or a mixture of carbon monoxide and any other non-oxidizing gas such as nitrogen or argon, in a suitable solvent in the presence of a catalyst. By carrying out the reaction in an alcohol the corresponding carbamate derivative can be obtained. This reaction of alcohols with isocyanates is well known. Alternatively, pure isocyanate may be synthesised by using very dry solvents that are inert to the isocyanate.

The organic nitro compound can be nitrobenzene or 2,4-dinitrotoluene. Low conversions and poor selectivity are encountered with 2,4-dinitrotoluene. With nitrobenzene however the conversion and the selectivity are much higher.

The pressure and temperature of the reaction influence the overall yield of the isocyanate derivative. Temperatures from 30°C to 200°C and pressures from atmospheric (101 kPa) to 5000 p.s.i. (34500 kPa) have been used. The optimum pressure and temperature are about 100 ps.i. (689 kPa) and 140°C.

The reaction can be conveniently carried out in a solvent such as tetrahydrofuran, acetonitrile, methanol, ethanol, propanol, isopropanol or butanol or in an aromatic solvent such as benzene or toluene. Alternatively, the nitro compound itself can be used as a solvent. Tetrahydrofuran, alcohols and acetonitrile

give better conversions and selectivities than the other solvents. The presence of water in the solvent causes the formation of amine and urea derivatives. Thus from nitro benzene, both aniline and diphenyl urea can be obtained in good yield by carrying out the direct carbonylation in wet tetrahydrofuran.

The catalyst is a polynuclear metal carbonyl or derivative thereof of the following general formula: $M_x(CO)_yL_z{}^{n-}$, where

| | | | | | |
|---|---|---|---|---|---|
| M = Fe | x = 3 | y = 12 | z = 0 | n = 0 | |
| Fe | x = 3 | y = 11 | z = 1 | L = H | n = 1 |
| Ru | x = 3 | y = 12 | z = 0 | n = 0 | |
| Ru | x = 3 | y = 11 | z = 1 | L = H | n = 1 |
| Ru | x = 4 | y = 12 | z = 4 | L = H | n = 0 |
| Rh | x = 6 | y = 16 | z = 0 | n = 0 | |
| Pt | x = 15 | y = 30 | z = 0 | n = 2 | |

For polynuclear metal carbonyl anions, i.e. cases where n = 0, the counter ion is a tetra-alkyl ammonium group. The catalytic activities of the polynuclear carbonyls vary widely and the ruthenium derivatives, especially $Ru_3(CO)_{12}$, are found to be the most active. With iron, platinum and rhodium derivatives, fairly rapid de-activation of the catalyst takes place. It is also possible to use a precursor such as ruthenium trichloride trihydrate as the catalyst which under the reaction conditions is converted partially into $Ru_3(CO)_{12}$.

The following Examples illustrate the invention.

<u>Example 1</u>

In an autoclave 100 parts of tetrahydrofuran (not dried) and 100 parts of nitrobenzene were reacted with two parts of triruthenium dodecacarbonyl under an initial pressure of 100 p.s.i. (689 kPa) of CO. The

mixture was heated at 140°C for six hours. The product mixture on gas chromatographic analysis showed the formation of 15 parts of phenyl isocyanate, 10 parts of aniline and 25 parts of diphenyl urea.

Example 2

In an autoclave, 100 parts of carefully dried acetonitrile and 100 parts of nitrobenzene were reacted with 2 parts of triruthenium dodecacarbonyl under an initial pressure of 200 p.s.i. (1380 kPa) of CO. The mixture was heated at 160°C for six hours. The product mixture on gas chromatographic analysis showed the formation of 70% of phenyl isocyanate.

Example 3

In autoclave 100 parts of dry ethanol and 100 parts of nitrobenzene were reacted with 2 parts of triruthenium dodecacarbonyl under an initial pressure of 150 p.s.i. (1034 kPa) of CO. The mixture was treated at 160°C for six hours. The product mixture on gas chromatographic analysis showed the formation of 65% of $PhNH-CO_2Et$.

Example 4

In an autoclave 100 parts of dry tetrahydrofuran and 100 parts of nitrobenzene were reacted with 2 parts of hexa-rhodium hexadecacarbonyl under an initial pressure of 200 p.s.i. (1380 kPa) of CO. The mixture was heated at 140°C for six hours. The product mixture on gas chromatographic analysis showed the formation of less than 5% phenyl isocyanate.

Example 5

In an autoclave 100 parts of dry ethanol and 100 parts of nitrobenzene were reacted with 2 parts of ruthenium trichloride trihydrate under an initial pressure of 150 p.s.i. (1034 kPa) of CO. The product mixture on gas chromatographic analysis showed the formation of 10% of $Ph-NH-CO_2Et$.

Example 6

In an autoclave 100 parts of dry tetrahydrofuran and 100 parts of 2,4-dinitrotoluene were reacted with 2 parts of triruthenium dodecacarbonyl under an initial pressure of 150 p.s.i. (1034 kPa) of CO. The mixture was heated at 160°C for six hours. The product mixture on gas chromatographic analysis showed the formation of 12% of toluene 2,4-di-isocyanate.

## CLAIMS

1. A process for the preparation of isocyanates or derivatives thereof which comprises reacting nitrobenzene or 2,4-dinitrotoluene with carbon monoxide or a mixture of carbon monoxide and inert gas in the presence of as catalyst a polynuclear metal carbonyl or derivative thereof of the formula: $M_x(CO)_yL_z{}^{n-}$, wherein

| | | | | | |
|---|---|---|---|---|---|
| M = Fe | x = 3 | y = 12 | z = 0 | n = 0 | |
| Fe | x = 3 | y = 11 | z = 1 | L = H | n = 1 |
| Ru | x = 3 | y = 12 | z = 0 | n = 0 | |
| Ru | x = 3 | y = 11 | z = 1 | L = H | n = 1 |
| Ru | x = 4 | y = 12 | z = 4 | L = H | n = 0 |
| Rh | x = 6 | y = 16 | z = 0 | n = 0 | |
| Pt | x = 15 | y = 30 | z = 0 | n = 2 | |

2. A process as claimed in claim 1 wherein the reaction is conducted at a temperature of from 30°C to 200°C.

3. A process as claimed in claim 1 or 2, wherein the reaction is conducted at a pressure of from 1 atmosphere (101 kPa) to 5000 p.s.i. (34500 kPa).

4. A process as claimed in any one of claims 1 to 3 wherein the reaction is conducted in the presence of an alcohol as solvent which reacts with the isocyanate product to give a urethane.

5. A process as claimed in claim 4 wherein the alcohol used is methanol, ethanol, propanol, isopropanol or butanol.

6. A process as claimed in any one of claims 1 to 3 wherein the reaction is conducted in the presence of an inert liquid diluent.

7. A process as claimed in claim 6 wherein the said diluent is tetrahydrofuran, acetonitrile, diglyme, benzene, or toluene.

8. A process as claimed in any one of claims 1 to 7 wherein the catalyst used is triruthenium dodecacarbonyl, which is produced in situ from ruthenium trichloride trihydrate.

9. A process as claimed in any of claims 1 to 3 wherein the product isocyanate is converted by water present in the solvent into the corresponding amine.

10. A process as claimed in any one of claims 1 to 9 wherein the carbon monoxide is used in admixture with nitrogen or argon.

European Patent Office

# EUROPEAN SEARCH REPORT

0115660

Application number

EP 83 30 0413

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | FR-A-1 558 899 (OLIN MATHIESON CHEMICAL CORP.) * Pages 1-3 * | 1 | C 07 C 118/06 |
| Y | FR-A-2 192 096 (SUMITOMO) * Page 7 * | 1 | |
| Y | US-A-3 070 618 (F.E. DRUMMOND) * Claims 1,2 * | 1 | |
| Y | US-A-4 179 469 (T. IMAI) * Claims 14-16 * | 1 | TECHNICAL FIELDS SEARCHED (Int. Cl. 3) |
| Y | CHEMICAL ABSTRACTS, vol. 80, no. 7, 18th February 1974, page 312, no. 37265t, Columbus, Ohio, USA E. SAPPA et al.: "Reactions of dodecacarbonyltriruthenium with nitrobenzene and aniline" & J. ORGANOMETAL. CHEM. 1973, 61, 383-388 * Abstract * | 1 | C 07 C 118/00 |

The present search report has been drawn up for all claims

| Place of search | Date of completion of the search | Examiner |
|---|---|---|
| THE HAGUE | 27-09-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82